# EUROPEAN PATENT SPECIFICATION

(11) **EP 0 292 396 B1**
(45) Date of publication and mention of the grant of the patent: **09.03.1994**
(21) Application number: 88401222.0
(22) Date of filing: 19.05.1988
(51) Int. Cl.: A61M 1/36, A61M 1/18

(54) **Extracorporeal blood circulating apparatus**
Gerät für die extrakorporale Blutzirkulation
Appareil pour la circulation extracorporelle du sang

(30) Priority: 19.05.1987 JP 122243/87; 18.12.1987 JP 320760/87
(43) Date of publication of application: 23.11.1988
(73) Proprietor: TERUMO KABUSHIKI KAISHA, Tokyo 151 (JP)
(72) Inventor: Katsura, Yoshiro, Fuji-shi Shizuoka-ken (JP)
(74) Representative: Joly, Jean-Jacques

(56) References cited:
- EP-A- 0 089 748

## Description

### BACKGROUND OF THE INVENTION

The present invention relates to an extracorporeal blood circulating apparatus.

When a thoracic operation, for example, is to be carried out on a patient, an extracorporeal blood circulating circuit is established using an artificial lung in which the blood is circulated for an exchange of carbon dioxide and oxygen. The blood is controlled at a prescribed temperature by a heat exchanger, and therafter the gases are exchanged in the articifial lung. Then, the blood is temporarily stored in a blood reservoir for a steady supply of the blood. The blood is pumped into the patient under the operation at a constant pulse rate.

Blood reservoirs for use in extracorporeal blood circulation include a closed-type blood reservoir in the form of a soft bag for storing blood in an airtight condition and an open-type blood reservoir in the form of a hard housing for storing blood.

An example of a blood oxygenating apparatus that uses an airtight, flexible membrane type of reservoir is disclosed in EP-A-0 089 748. Here, the reservoir comprises a rigid concave shell on which the flexible membrane forms a liquid-tight variable volume. The inlet ports to the reservoir enter into the rear shell wall adjacent to, but spaced apart from the lower end of the reservoir. The open-type blood reservoir is advantageous in that priming and confirmation of the stored amount of blood can easily be performed, and it would be easy to construct the blood reservoir as a unitary component of an artificial lung. Therefore, various extracorporeal blood circulating apparatus employing open-type blood reservoirs have been proposed.

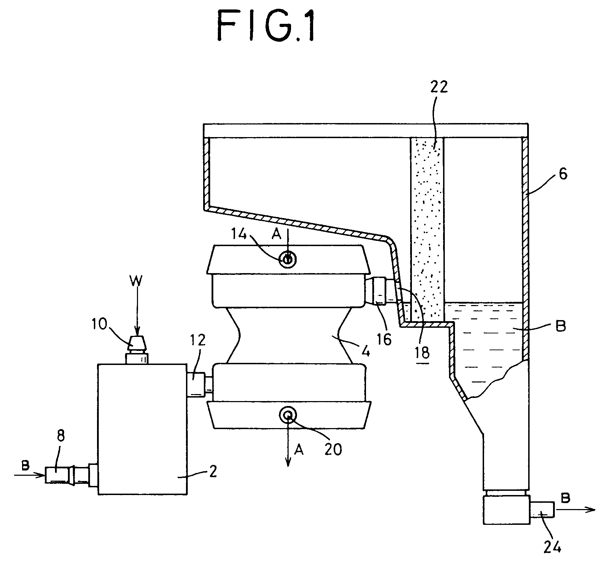

FIG. 1 of the accompanying drawings schematically illustrates a extracorporeal blood circulating apparatus proposed by the applicant. The extracorporeal blood circulating appartus comprises a heat exchanger 2, an artificial lung 4, and a blood reservoir 6 as an interconnected unitary system. The extracorporeal blood circulating apparatus is connected to the body of a patient through a pump (not shown) which supplies blood at a certain pulse rate. The extracorporeal blood circulating apparatus is positioned in a location lower than the patient's body. Blood B discharged from the human body is introduced under the pressure developed by the blood head from a blood inlet port 8 into the heat exchanger 2 and controlled at a prescribed temperature by warm or cold water supplied from a water inlet port 10. Thereafter, the blood B is fed through a joint tube 12 into the artificial lung 4. The artificial lung 4 contains a multiplicity of hollow filamentary membranes through which a gas A containing oxygen supplied from a gas inlet port 14 flows. The blood B, while passing around the hollow filamentary membranes, receives oxygen from the gas A and discharges carbon dioxide, and then flows via a joint tube 16 through a blood inlet port 18 into the blood reservoir 6. After the exchange of oxygen and carbon dioxide, the gas A is discharged from the artificial lung 4 through a gas outlet port 20. The blood B that has entered the blood reservoir 6 from the blood inlet port 18 goes through an anti-foaming member 22 and is temporarily stored in the blood reservoir 6. Then, the stored blood B is supplied from a blood outlet port 24 into the human body by the pump.

With the above extracorporeal blood circulating apparatus, the blood B is circulated between the apparatus and the human body by the pressure developed by the blood head and the pump. If the artificial lung 4 and the blood reservoir 6 are connected as shown in FIG. 1, since the joint tube 16 lies substantially horizontally, the blood B may gush into the blood reservoir 6 under the pressure developed by the blood head. The blood B stored in the blood reservoir 6 is supplied from the blood outlet port 24 into the human body under the pulse rate determined by the pump. The blood B may therefore tend to apply unwanted vibration to the apparatus.

### SUMMARY OF THE INVENTION

It is an objet of the present invention to provide an extracorporeal blood circulating apparatus capable of effecting a smooth influx of blood into a blood reservoir. According to the invention, the extracorporeal blood circulating apparatus comprises an antificial lung and a blood reservoir which are interconnected by a joint tube a first end of which is connected to the artificial lung, and a second end of which is connected to the blood reservoir. According to the present invention, the second end of the joint tube is connected to a bottom surface of the blood reservoir for delivering blood from said antificial lung upwardly into the blood reservoir. In this way, the generation of any unwanted vibration which would otherwise be applied to the apparatus can be controlled.

The joint tube may be substantially of an L shape including a horizontal portion extending from the artificial lung and a vertical portion extending upwardly from the horizontal portion over a predetermined length.

A blood dispersing member is preferably mounted in the end of the joint tube opening into the blood reservoir for smoothing the flow of the blood. The blood dispersing member may comprise a mesh screen.

The above and other features and advantages of the present invention will become more apparent from the following description when taken in conjunction with the accompanying drawings in which a preferred embodiment of the present invention is shown by way of illustrative example.

### BRIEF DESCRIPTION OF THE DRAWINGS

FIG. 1 is a schematic elevational view, partly in cross section, of an extracorporeal blood circulating apparatus shown as a comparative example ;
FIG. 2 is a schematic elevational view, partly in cross section, of a extracorporeal blood circulating apparatus according to the present invention ; and
FIG 3 is a schematic perspective view of the extracorporeal blood circulating apparatus illustrated in FIG. 2.

### DETAILED DESCRIPTION OF THE PREFERRED EMBODIMENT

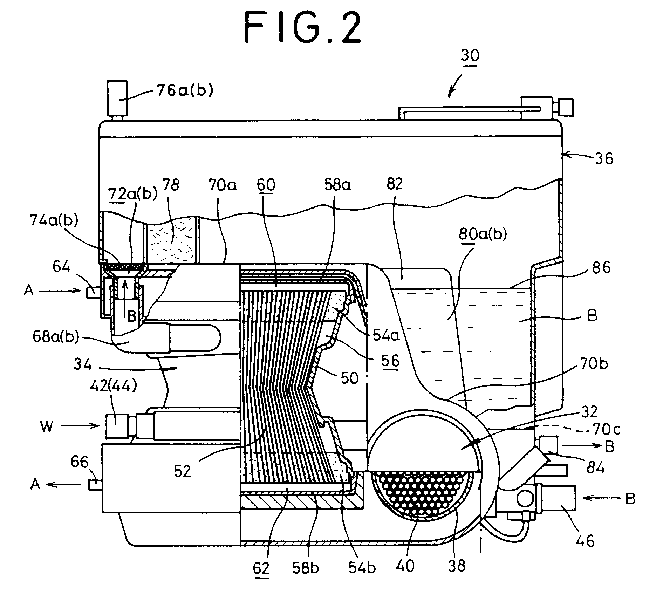

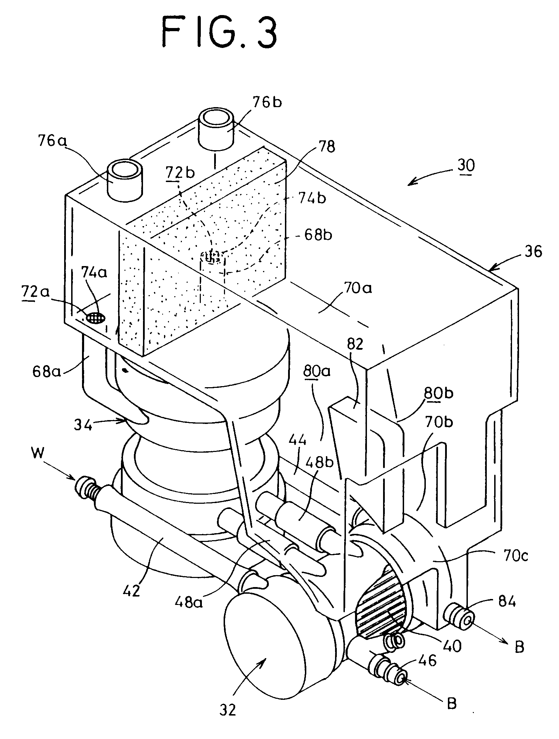

As shown in FIGS. 2 and 3, an extracorporeal blood circulating apparatus according to the present invention has a unitary apparatus assembly 30 comprising a heat exchanger 32, an artificial lung 34, and an open-type blood reservoir 36.

The heat exchanger 32 includes a tubular housing 38 accommodating therein a number of heat exchanger pipes 40. Warm or cold water W is supplied from a water port 42 to the pipes 40, and then discharged from the pipes 40 via a water port 44. Blood B flowing from a blood supply port 46 into the heat exchanger 32 is heated or cooled to a prescribed temperature while it is passing around the pipes 40. Thereafter, the blood B is supplied via two joint tubes 48a, 48b (see FIG. 3) to the artificial lung 34.

The artificial lung 34 serves to remove carbon dioxide from the blood B and add oxygen to the blood B. The artificial lung 34 includes a multiplicity of hollow filamentary membranes 52 bundled and stored in a substantially cylindrical housing 50 with a constricted central portion. On the upper and lower ends of the housing 50, there are mounted partitions 54a, 54b holding the opposite ends of the hollow filamentary membranes 52. The housing 50 and the partitions 54a, 54b jointly define a space surrounded thereby and housing the hollow filamentary membranes 52 therein, the space having a lower end communicating with the heat exchanger 32 through the joint tubes 48a, 48b. This space serves as a gas exchanging chamber 56 through which the blood B flows. Covers 58a, 58b are also mounted on the upper and lower ends of the housing 50. The partition 54a and the cover 58a define a gas supply chamber 60 therebetween, and the partition 54b and the cover 58b define a gas discharge chamber 62 therebetween. The hollow filamentary membranes 52 are supplied with a gas A containing oxygen from a gas inlet port 64 via the gas supply chamber 60. The gas A delivered from the hollow filamentary membranes 52 is discharged through the gas discharge chamber 62 from a gas outlet port 66.

The gas exchanging chamber 56 in the artificial lung 34 has an upper end communicating with the blood reservoir 36 through two L-shaped joint tubes 68a, 68b each having a horizontal portion extending from the gas exchanging chamber 56 and a vertical portion extending upwardly from the end of the horizontal portion over a certain length. The blood reservoir 36 has a bottom plate composed of first through third steps 70a, 70b, 70c. The joint tubes 68a, 68b have blood inlet ports 72a, 72b, respectively, opening at the first uppermost step 70a on its lateral sides through blood dispersing mesh screens 74a, 74b, respectively, for smoothing the blood flow flowing therethrough. The blood reservoir 36 is made of a transparent plastic material. The blood reservoir 36 has a pair of drug inlet ports 76a, 76b above the blood inlet ports 72a, 72b, respectively, for adding various drugs such as vasodilatator drug, an anticoagulant, and the like to the blood B.

A urethane anti-foaming member 78 is disposed on the first step 70a of the blood reservoir 36 near the blood inlet ports 72a, 72b. A partition 82 is mounted substantially centrally on the second step 70b and divides the blood storage space on the second step 70b into a first blood storage region 80a and a second blood storage region 80b for preventing the blood surface from being subjected to resonant vibration. The third lowermost step 70c is coupled to a blood outlet port 84 for supplying the blood B stored in the blood reservoir 36 into the human body. In operation, the blood outlet port 84 is coupled to the human body through a pump (not shown) which delivers the blood at a prescribed pulse rate. In the case, the pump may be a rotary type or peristaltic finger type.

The extracorporeal blood circulating apparatus of the present invention is basically of the above structure. Now, operation and advantages of the extracorporeal blood circulating apparatus will be described below.

The blood B coming from the blood supply port 46 is supplied through the gaps between the tubes 40 stored in the heat exchanger 32 to the artificial lung 34. At this time, water W maintained at a prescribed temperature is flowing through the heat exchanger tubes 40 through the water ports 42, 44. The blood B is heater or cooled to a predetermined temperature by the water W flowing through the tubes 40.

The blood B fed from the heat exchanger 32 via the joint tubes 48a, 48b into the artificial lung 34 enters the gas exchanging chamber 56 surrounded by the housing 50 in which oxygen is added to the blood B and carbon dioxide is removed from the blood B. More specifically, the gas A containing oxygen is supplied into the bundled hollow filamentary membranes 52 in the housing 50 from the gas inlet port 64 through the gas supply chamber 60. Oxygen and carbon dioxide are exchanged in the blood B through the hollow filamentary membranes 52. The gas A containing carbon dioxide is discharged via the gas discharge chamber 62 from the gas outlet port 66.

The blood B to which oxygen has been added in the artificial lung 34 then flows through the joint tubes 68a, 68b and the blood inlet ports 72a, 72b into the blood reservoir 36. As described above, the joint tubes 68a, 68b are of the L shape including a horizontal portion extending from the upper end of the gas exchanging chamber 56 ans a vertical portion extending upwardly from the horizontal portion over a certain length. Therefore, the blood B flowing out of the gas exchanging chamber 56 under the pressure developed by the pump (not shown) or the blood head produced by the higher human body position flows upwardly through the L-shaped joint tubes 68a, 68b, during which time the kinetic energy of the blood B is reduced by an increase in the potential energy thereof, thereby resulting in a reduction in the speed of flow of the blood B. As a consequence, the blood B is allowed to flow smoothly into the blood reservoir 36, without the danger of unwanted vibration which would otherwise be applied to the apparatus by the pressure under which the blood B would flow into the blood reservoir 36. The blood B is dispersed by the mesh screens 74a, 74b mounted respectively in the blood inlet ports 72a, 72b, and then stored into the blood reservoir 36 through the urethane anti-foaming member 78.

The blood B stored in the blood reservoir 36 is then intermittently or pulsatively delivered out to the human body by the pump (not shown) coupled to the blood outlet port 84. The surface level 86 of the blood B stored in the blood reservoir 36 would be subjected to resonant vibration due to intermittent delivery of the blood B by the pump. However, such resonant vibration is highly effectively suppressed by the partition 82 on the second step 70b in the blood reservoir 36.

More specifically, it is considered that resonant vibration which would otherwise be caused on the surface level 86 of the blood B stored in the blood reservoir 36 would be produced when the natural frequency of the blood storage system based on the characteristics of the blood B, the configuration of the blood reservoir 36, and other parameters coincides with the pulse rate of the pump. The pulse rate of the pump is selected to be in a certain range for practical reasons with respect to the artificial lung 34. The partition 82 located in the blood reservoir 36 is effective in making the system's natural frequency widely different from the pulse rate of the pump, thus suppressing the undesirable resonant vibration. Therefore, the surface level 86 of the blood B is kept calm without violent turbulent motion. Since the fluctuation of the surface level 86 arising from the pulse rate of the pump is small, the amount of the blood B stored in the blood reservoir 36 can easily and accurately be checked by a visual inspection. Moreover, inasmuch as any turbulent motion of the blood B in the blood reservoir 36 is effectively suppressed, vibration of the blood B is held to a minimum, and no unwanted vibration is applied to the apparatus.

With the present invention, as described above, the extracorporeal blood circulating apparatus includes the artificial lung and the blood reservoir which are joined to each other, and the blood flowing out of the artificial lung is caused to flow upwardly through the joint tubes including upwardly extending portions onto the bottom of the blood reservoir. Since the speed of flow of the blood from the artificial lung is effectively reduced while flowing upwardly through the joint tubes, the blood is allowed to flow smoothly into the blood reservoir. As a result, the pressure under which the blood flows into the blood reservoir is lowered, thereby preventing unwanted pressure from being imposed on the apparatus.

The present invention is not limited to the illustrated embodiment, but the principles of the invention are also applicable to a extracorporeal blood circulating apparatus which is separate from a heat exchanger, for example.

## Claims

1. An extracorporeal blood circulating apparatus comprising an artificial lung (34) and an open type blood reservoir (36) which are interconnected by at least one joint tube (68a, 68b), a first end of said at least one joint tube (68a, 68b) being connected to said artificial lung (34), and a second end of said at least one joint tube being connected to said blood reservoir (36),
characterized in that said second end of said at least one joint tube (68a, 68b) is connected to a bottom surface of said blood reservoir (36) for delivering blood from said artificial lung upwardly into said blood reservoir.

2. An extracorporeal blood circulating apparatus according to claim 1, wherein said at least one joint tube (68a, 68b) is substantially of an L shape including a horizontal portion extending from said artificial lung and a vertical portion extending upwardly from said horizontal portion over a predetermined length.

3. An extracorporeal blood circulating apparatus according to claim 1, further including blood flow impeding member (74a, 74b) mounted in an end of said at least one joint tube opening into said blood reservoir (36) for smoothing the flow of the blood.

4. An extracorporeal blood circulating apparatus according to claim 3, wherein said blood flow impeding member comprises a mesh screen.

## Patentansprüche

1. Gerät für die extrakorporale Blutzirkulation mit einer künstlichen Lunge (34) und einem Blutbehälter (36) offener Bauart, welche über zumindest eine Verbindungsröhre (68a, 68b) miteinander verbunden sind, wobei ein erstes Ende der zumindest einen Verbindungsröhre (68a, 68b) mit der künstlichen Lunge (34) und ein zweites Ende der zumindest einen Verbindungsröhremit mit dem Blutbehälter (36) verbunden ist,
dadurch gekennzeichnet, daß das zweite Ende der zumindest einen Verbindungsröhre (68a, 68b) mit einer Bodenfläche des Blutbehälters (36) verbunden ist, um das Blut von der künstlichen Lunge nach oben in den Blutbehälter zu befördern.

2. Gerät für die extrakorporale Blutzirkulation nach Anspruch 1, wobei die zumindest eine Verbindungsröhre (68a, 68b) im wesentlichen L-förmig ist und ein waagrechtes Stück, das sich von der künstlichen Lunge aus erstreckt, und einen senkrechtes Stück hat, das sich nach oben über eine vorbestimmte Länge vom waagrechten Stück erstreckt.

3. Gerät für die extrakorporale Blutzirkulation nach Anspruch 1, das ferner ein Teil (74a, 74b) zur Hemmung des Blutflusses hat, das in einem Ende der zumindest einen Verbindungsröhre sitz und in den Blutbehälter (36) öffnet, um den Blutfluß zu glätten.

4. Gerät für die extrakorporale Blutzirkulation nach Anspruch 3, bei dem das Teil zur Hemmung des Blutflusses ein Maschensieb ist.

## Revendications

1. Appareil pour la circulation extra-corporelle du sang comportant un poumon artificiel (34) et un réservoir de sang du type ouvert (36) qui sont interconnectés par au moins un tube de liaison (68a, 68b), une première extrémité dudit tube de liaison (68a, 68b) étant reliée audit poumon artificiel (34), et une deuxième extrémité dudit tube de liaison étant reliée audit réservoir de sang (36),
caractérisé en ce que ladite deuxième extrémité dudit tube de liaison (68a, 68b) est reliée à une surface inférieure dudit réservoir de sang (36) afin de délivrer du sang vers le haut dans ledit réservoir de sang depuis ledit poumon artificiel.

2. Appareil pour la circulation extra-corporelle du sang selon la revendication 1, dans lequel ledit tube de liaison (68a, 68b) est sensiblement en forme de L comprenant une partie horizontale s'étendant depuis ledit poumon artificiel et une partie verticale s'étendant vers le haut depuis ladite partie horizontale sur une longueur prédéterminée.

3. Appareil pour la circulation extra-corporelle du sang selon la revendication 1, comportant en outre un élément freinant l'écoulement du sang (74a, 74b) monté dans une extrémité de ladite ouverture de tube de liaison dans ledit réservoir de sang (36) afin de rendre régulier l'écoulement du sang.

4. Appareil pour la circulation extra-corporelle du sang selon la revendication 3, dans lequel ledit élément freinant l'écoulement du sang comprend une grille.
